# FASCICULE DE BREVET EUROPEEN

(11) **EP 2 794 012 B1**
(45) Date de publication et mention de la délivrance du brevet: **16.09.2015**
(21) Numéro de dépôt: 12815742.7
(22) Date de dépôt: 14.12.2012
(51) Int. Cl.: A61Q 5/00, A61Q 5/02, A61K 8/73, A61K 8/97, A61K 31/737

(54) **UTILISATION DE POLYSACCHARIDES SULFATÉS COMME AGENT ANTIPELLICULAIRE**
VERWENDUNG VON SULFATIERTEN POLYSACCHARIDEN ALS ANTISCHUPPENMITTEL
USE OF SULPHATED POLYSACCHARIDES AS ANTIDANDRUFF AGENT

(30) Priorité: 20.12.2011 FR 1162109; 22.12.2011 US 201161579038 P
(43) Date de publication de la demande: 29.10.2014
(73) Titulaire: L'Oréal, 75008 Paris (FR)
(72) Inventeur: POTTER, Anne, F-92200 Neuilly- Sur- Seine (FR); THIBAUT, Sébastien, F-92800 Puteaux (FR); RIBAUT, Christèle, F-94130 Nogent S/marne (FR)
(74) Mandataire: Kromer, Christophe
(86) Numéro de dépôt international: PCT/FR2012/052936
(87) Numéro de publication internationale: WO 2013/093307

(56) Documents cités:
- EP-A2- 0 965 323
- WO-A1-98/10656
- FR-A- 1 596 818
- FR-A1- 2 882 258
- US-A1- 2011 021 457

## Description

La présente invention concerne l'utilisation de polysaccharides sulfatés particuliers comme agent antipelliculaire, en particulier dans le traitement cosmétique des états pelliculaires liés à la prolifération excessive des levures du genre Malassezia sur le cuir chevelu. L'invention concerne également un procédé de traitement cosmétique destiné à éliminer et/ou réduire les pellicules, notamment celles provoquées par les levures du genre Malassezia, employant lesdits polysaccharides sulfatés.

L'apparition de pellicules, correspondant à un désordre desquamatif du cuir chevelu, est gênante tant du point de vue esthétique que des désagréments qu'elle provoque (démangeaisons, rougeurs, etc.), si bien que beaucoup de gens confrontés à ce problème à des degrés variables souhaitent s'en débarrasser de manière efficace et définitive.

Les pellicules correspondent à une desquamation excessive et visible du cuir chevelu, résultant de la multiplication trop rapide des cellules épidermiques. Ce phénomène peut être provoqué notamment par des microtraumatismes de nature physique ou chimique, tels que des traitements capillaires trop agressifs, des conditions climatiques extrêmes, la nervosité, l'alimentation, la fatigue, la pollution; mais il a été démontré que les états pelliculaires résultent le plus souvent d'un désordre de la microflore du cuir chevelu, et plus particulièrement de la colonisation excessive d'une levure qui appartient à la famille des levures du genre Malassezia (autrefois appelées Pytirosporum ovale) et qui est naturellement présent sur le cuir chevelu.

Pour lutter contre les pellicules, il est connu d'utiliser des agents anti-fongiques appliqués par voie topique. Ces agents visent, par leur pouvoir anti-fongique, à éliminer ou contrôler la multiplication d'une levure résidente du cuir chevelu, appartenant au genre *Malassezia* et ses variantes (*M*. *ovalis, M. orbiculare, M. furfur, M. globosa*...).

De nombreux agents sont revendiqués, connus et utilisés dans cet effet, parmi lesquels on peut citer, le Zinc Pyrithione, La Piroctone Olamine, le Disulfure de Sélenium. Toutefois, ces agents antipelliculaires ne sont pas totalement satisfaisants en terme d'efficacité (efficacité immédiate ou durée de l'effet) et/ou en terme d'impact sur l'environnement.

La présente invention a pour but de proposer des agents antipelliculaires non irritants pour la peau et le cuir chevelu, aussi efficaces que les agents antipelliculaires connus, tout en ayant un impact plus favorable en terme d'environnement (faible bioaccumulation et bonne biodégradabilité notamment).

L'invention a également pour but de proposer des actifs permettant de prévenir la colonisation excessive du cuir chevelu par Malassezia sp, et en particulier de limiter le désordre desquamatif lié au métabolisme des Malassezia.

La demanderesse a maintenant trouvé de manière surprenante que l'utilisation de certains polysaccharides sulfatés tels que définis ci-après permettent de traiter efficacement les états pelliculaires, notamment ceux associés à la prolifération de levures du genre Malassezia, et de remédier aux inconvénients de l'art antérieur.

Il a été observé qu'en employant les polysaccharides sulfatés définis ci-après, on pouvait éliminer et/ou réduire le nombre de pellicules, et le désordre desquamatif engendré par les Malassezia et leurs métabolites, comme l'acide oléique.

La présente invention a donc pour objet l'utilisation cosmétique de polysaccharide sulfaté particulier tels que définis ci-après comme actif pour prévenir et/ou traiter les pellicules du cuir chevelu.

L'invention a également pour objet un procédé de traitement cosmétique pour prévenir et/ou traiter les pellicules, notamment celles provoquées par les levures du genre Malassezia, caractérisé en ce qu'il comprend l'application, sur le cuir chevelu, d'au moins un polysaccharide sulfaté tel que défini ci-après.

Avantageusement, l'utilisation et le procédé selon l'invention sont mis en oeuvre pour traiter les pellicules dues à la prolifération de levures du genre Malassezia sur le cuir chevelu.

Les polysaccharides sulfatés utilisés selon l'invention sont choisis parmi le dextrane sulfate et les polysaccharides sulfatés issus de l'algue rouge Porphyridium sp.

Avantageusement, le taux de sulfatation des polysaccharides peut aller de 1 % à 30 % en poids, par rapport au poids du polysaccharide. De préférence ce taux de sulfatation peut aller de 2 % à 25 % en poids.

Le polysaccharide peut être éventuellement acétylé. Le taux d'acétylation peut aller de 0 à 10 % en poids (taux pondéral de motifs acétyle par rapport au poids total du polymère) .

Le polysaccharide utilisé selon l'invention a de préférence un poids moléculaire moyen en en poids est compris entre 1000 et 20.10⁶ Daltons, préférentiellement entre 2000 et 10.10⁶ Da, préférentiellement entre 5 000 et 5.10⁶ Da et plus préférentiellement entre 10 000 et 1.10⁶ Da.

Le polysaccharide sulfaté utilisé selon l'invention peut être le dextran sulfate .On peut utiliser le dextran sulfate vendu sous la dénomination commerciale « Dextran sulfate 10 sodium salt CG » par la société PK chemicals.

Le polysaccharide sulfaté utilisé selon l'invention peut être choisi parmi les polysaccharides sulfatés issus de l'algue rouge Porphyridium sp , tel que celui vendu sous la dénomination « Alguard® » par la société Frutarom. La demande EP-A-311496 décrit notamment un procédé de préparation de tels polysaccharides. Ces polysaccharides sulfatés contiennent de l'acide glucuronique, du xylose, du glucose, du galactose sulfatés.

Le polysaccharide sulfaté utilisé selon l'invention est avantageusement présent dans une composition cosmétique contenant un milieu physiologiquement acceptable.

On entend par milieu physiologiquement acceptable, un milieu compatible avec les tissus cutanés tels que la peau et le cuir chevelu.

Le polysaccharide sulfaté utilisé selon l'invention peut être présent dans la composition cosmétique en une teneur allant de 0,01 à 20 % en poids, par rapport au poids total de la composition cosmétique, de préférence allant de 0,05 à 15 % en poids, préférentiellement allant de 0,1 à 10 % en poids, et plus préférentiellement allant de 0,1 à 5 % en poids.

Le milieu physiologiquement acceptable de la composition peut être plus particulièrement constitué d'eau et éventuellement d'un solvant organique physiologiquement acceptable choisi par exemple parmi les alcools inférieurs comportant de 2 à 8 atomes de carbone et en particulier de 2 à 6 atomes de carbone, comme l'éthanol, l'isopropanol, le propanol, le butanol ; les polyéthylène glycols ayant de 6 à 80 unités oxyde d'éthylène et les polyols comme le propylène glycol, l'isoprène glycol, le butylène glycol, la glycérine et le sorbitol.

Les compositions selon l'invention peuvent se présenter sous toutes les formes galéniques classiquement utilisées pour une application topique et notamment sous forme de solutions aqueuses, hydroalcooliques, d'émulsions huile-dans-eau (H/E) ou eau-dans-huile (E/H) ou multiple (triple : E/H/E ou H/E/H), de gels aqueux, ou de dispersions d'une phase grasse dans une phase aqueuse à l'aide de sphérules, ces sphérules pouvant être des nanoparticules polymériques telles que les nanosphères et les nanocapsules ou des vésicules lipidiques de type ionique et/ou non ionique (liposomes, niosomes, oléosomes). Ces compositions sont préparées selon les méthodes usuelles.

En outre, les compositions utilisées selon l'invention peuvent être plus ou moins fluides et avoir l'aspect d'une crème blanche ou colorée, d'une pommade, d'un lait, d'une lotion, d'un sérum, d'une pâte ,d'une mousse, de shampooing.

La composition utilisée selon l'invention comprendre des adjuvants usuellement employés dans le domaine cosmétique, et notamment choisis parmi l'eau; les huiles ; les cires, les pigments, les charges, les colorants, les tensioactifs, les émulsionnants ; les actifs cosmétiques, les filtres UV, les polymères, les épaississants, les polymères filmogènes, les conservateurs, les parfums, les bactéricides , les absorbeurs d'odeur, les antioxydants.

Les quantités de ces différents adjuvants sont celles classiquement utilisées dans le domaine considéré, et par exemple de 0,01 à 20 % du poids total de la composition.

Les exemples ci-après illustrent l'invention sans toutefois en limiter la portée.

### Exemple 1 : Evaluation de l'action antipelliculaire des Polysaccharides sulfatés

### Protocole

Mise en évidence de la régulation de l'homéostasie épidermique par les polysaccharides sulfatés sur modèle de peau reconstruite modifiée par un métabolite de la flore du scalp

L'effet de l'application de polysaccharides sulfatés a été évalué dans un modèle de peau reconstruite présentant un désordre de l'homéostasie cutanée générant l'apparition d'agrégats analogues à des pellicules.

Ce désordre est obtenu par l'application topique (pendant 48 heures) d'une solution propylène glycol / éthanol / eau (proportions en volume 5/60/35) contenant 5 % en poids d'acide oléique. Cette méthode est décrite dans la littérature pour entraîner la formation de pellicules sur la surface de la peau humaine (Y. M. DeAngelis et al., Three Etiologic Facets of Dandruff and Seborrheic Dermatitis: Malassezia Fungi, Sebaceous Lipids, and Individual Sensitivity. J Investig Dermatol Symp Proc 10:295 -297, 2005). De la même façon, l'application d'acide oléique sur des échantillons de peaux reconstruites entraîne un déséquilibre de l'homéostasie cutanée (hyper-prolifération cellulaire et défaut de différenciation terminale) et provoque la formation d'agrégats en surface appelées « pseudo-pellicules », proches des pellicules observées sur un cuir chevelu.

### a) Réception des peaux

Les peaux reconstruites, produites par la société Episkin, se présentent en plaques contenant un milieu de croissance gélifié (GDA3F de chez Episkin). A réception, les peaux ont été placées dans de nouvelles boites « deep-well » contenant 8,5 ml de milieu GDA3F (liquide), puis mises dans un incubateur à 37°C dont l'atmosphère est saturée en eau et contrôlée en CO₂ (5%).

### b) 5 Conditions étudiées sur peau :

- 1 peau « témoin » : la peau n'a reçu aucun traitement
- 1 peau « contrôle véhicule »
- 2 peaux « altérées par l'acide oléique »
- 2 peaux « contrôle polysaccharide sulfaté »
- 2 peaux « altérées par l'acide oléique + application de polysaccharide sulfaté»

### c) Traitements

après 20 jours : Renouvellement de l'ensemble des milieux (8,5 ml)
   La peau « témoin » n'a reçu aucun traitement. Sur chacune des autres peaux, ont été appliquées 25 µL de solutions. Le milieu véhicule est un mélange de propylène glycol / éthanol / eau (5/60/35). Le poly-saccharide sulfaté est appliqué à 2 % en poids dans le milieu véhicule.
Après 22 jours : Arrêt des essais.
   Prélèvements des différents échantillons de peaux. Les échantillons de peaux ont été photographiés à l'aide d'un appareil Nikon 5000D puis prélevés à l'aide d'une presse à cran (découpe punch de taille 12 mm), puis inclus en tissue-TEK.

L'expérience a été renouvelée sur 3 lots de peaux différents.

### d) Méthodes d'analyses des résultats et scoring.

### Observations macroscopiques de la surface des échantillons et scoring de l'état pseudo-pelliculaire de la peau reconstruite

Afin d'évaluer l'état de surface des échantillons, les peaux reconstruites photographiées ont été observées et l'état pseudo-pelliculaire a été scoré à l'aide d'une échelle de mesure (table ci-dessous) définissant l'état pseudo-pelliculaire en fonction de la surface de l'échantillon recouverte par les pseudo-pellicules.

| Etat de surface observé | Score attribué |
|---|---|
| Surface intacte | 0 |
| Surface recouverte par quelques zones pseudopelliculaires | 1 |
| Surface recouverte par plus de la moitié de pseudo-pellicules | 2 |
| Surface recouverte en totalité par des pseudos-pelliculles | 3 |

### Résultats

### Exemple 1a : polydextran sulfaté

Le polysaccharide testé est le dextran sulfate, vendu sous la référence commerciale « Dextran sulfate 10 sodium salt CG » par la société PK Chemicals.

| *Conditions testées sur peau* | *Score Pseudo-pelliculaire* |
|---|---|
| Témoin | Score 0 |
| Contrôle Véhicule | Score 0 |
| Contrôle Polydextran Sulfaté | Score 0 |
| Application Acide Oléique | Score 3 |
| Application Polydextran Sulfaté + Application Acide Oléique | Score 0 |

### Exemple 1b : Polysaccharide issu de porphyridium

Le polysaccharide issu de Porphyridium est vendu sous la dénomination Alguard® par la société Frutarom.

| *Conditions testées sur peau* | *Score Pseudo-pelliculaire* |
|---|---|
| Témoin | Score 0 |
| Contrôle Véhicule | Score 0 |
| Contrôle porphyridium Alguard | Score 0 |
| Application Acide Oléique | Score 3 |
| Application porphyridium Alguard + Application Acide Oléique | Score 0 |

### Exemple 1c : Carraqhenane (hors invention)

On a évalué 2 carraghénanes différents :
Carraghénane 1 : vendu sous la dénomination SATIAGUM UTC 30 par la société Cargill
Carraghénane 2 : vendu sous la dénomination SATIAGUM UTC 10 par la société Cargill

| *Conditions testées* | *Score Pseudo-pelliculaire* |
|---|---|
| Témoin | Score 0 |
| Contrôle Véhicule | Score 0 |
| Contrôle Carraghenane 1 | Score 0 |
| Application Acide Oléique | Score 3 |
| Application Carraghenane 1 + Application Acide Oléique | Score 1 |
| Témoin | Score 0 |
| Contrôle Véhicule | Score 0 |
| Contrôle Carraghenane 2 | Score 0 |
| Application Acide Oléique | Score 3 |
| Application Carraghenane 2 + Application Acide Oléique | Score 2 |

### Conclusions

Les résultats obtenus montrent que le traitement de la peau avec le dextran sulfate, le polysaccharide issu de Porphyridium ont une action antipelliculaire efficace : aucun pseudo-pellicule n'est observé à la surface des échantillons de peau traités par ces polysaccharides sulfatés.

L'application des carraghenanes 1 et 2 ne permettent pas d'inhiber totalement l'apparition des « pseudo-pellicules » en surface des échantillons de peau reconstruite traités. Ils n'ont pas d'action antipelliculaire efficace.

### Exemple 2 : détermination de l'absence d'activité antifongique sur la souche Malassezia

Préparation de la solution de polysaccharide sulfaté testé à 1 % en poids dans du Leeming et Notman agar modifié ( LNm)

Les solutions initiales des produits à tester sont deux fois plus concentrées que la concentration finale de test pour tenir compte de la dilution au ½ lors de la mise en contact avec la suspension de Malassezia.

Le test est effectué sur les souches Malassezia globosa et Malassezia restricta.

Mettre en contact les souches Malassezia suivant le tableau ci dessous :

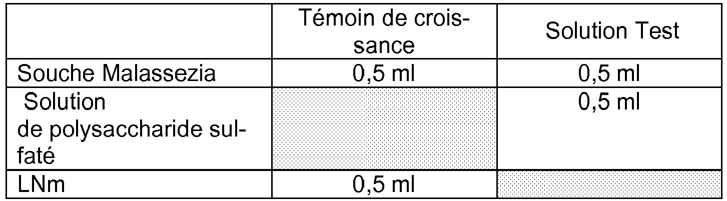

Agiter et déposer à la surface de la gélose LNm le mélange. Etaler avec un râteau stérile sur toute la surface avant de récupérer l'excédent.
Incuber au moins 5 jours à 30°C.

L'effet antifongique du polysaccharide sulfaté testé a été évalué par l'absence de croissance de la souche Malassezia testée, par rapport au témoin de croissance.

Les inhibitions ont été notées de 3 à 0 par appréciation de la densité de la culture à la surface de la gélose en comparaison avec le témoin de croissance de la souche

| **NOTATION** | **INHIBITION** | **INTERPRÉTATION** |
|---|---|---|
| 3 | 100% | Pas de croissance |
| 2 | 75% | Croissance < à la boîte témoin |
| 1 | 25% | Croissance < à la boîte témoin |
| 0 | 0% | Croissance comparable à la boîte témoin |

On a obtenu les résultats suivants :

| % Inhibition | | **Malassezia restricta** | **Malassezia globosa** |
|---|---|---|---|
| **Témoin de croissance** | | *Culture dense* | *Culture dense* |
| **Alguard®** | *1* % | *0* | *0* |

Les résultats obtenus montrent que le polyssaccharide testé (Alguard®) ne présente pas d'activité antifongique sur les espèces Malassezia globosa et restricta.

### Exemple 3 : shampooing antipelliculaire

On prépare un shampooing antipelliculaire comprenant les ingrédients suivants :

| | |
|---|---|
| Lauryl éther sulfate de sodium (2.2 OE) en solution aqueuse (TEXAPON AOS 225 UP de COGNIS) | 17 g MA |
| Cocoyl bétaine en solution aqueuse (DEHYTON AB 30 de COGNIS) | 2,5 g MA |
| Monoisopropanolamide d'acides de coprah (REWOMID V 3203 de GOLDSCHMIDT) | 2,0 g |
| Dextran sulfate | 0,3 g MA |
| Conservateurs | 1,1 g |
| Parfum | 0,5 |
| Eau | QS 100 g |

Le shampoing appliqué sur les cheveux et le cuir chevelu permet d'atténuer l'apparition des pellicules.

### Exemple 4 : lotion antipelliculaire

On prépare une lotion antipelliculaire comprenant les ingrédients suivants :
- Polysaccharide sulfaté issu de l'algue rouge Porphyridium sp (« Alguard® » de chez Frutarom) 0,3 g MA
- Conservateurs qs
- Eau qsp 100 g

La lotion appliquée sur les cheveux et le cuir chevelu permet d'atténuer l'apparition des pellicules.

## Revendications

1. Utilisation cosmétique de polysaccharide sulfaté choisi parmi le dextran sulfate, les polysaccharides sulfatés issus de l'algue rouge Porphyridium sp comme actif pour prévenir et/ou traiter les pellicules du cuir chevelu.

2. Utilisation selon la revendication 1 pour traiter les états pelliculaires associés à la prolifération de levures du genre Malassezia sur le cuir chevelu.

3. Utilisation selon l'une quelconque des revendications précédentes, **caractérisée en ce que** le polysaccharide sulfaté est le dextran sulfate.

4. Utilisation selon l'une quelconque des revendications 1 ou 2, **caractérisée en ce que** le polysaccharide sulfaté est issu de l'algue rouge Porphyridium sp.

5. Procédé de traitement cosmétique destiné à éliminer et/ou réduire les pellicules, notamment celles provoquées par les levures du genre Malassezia, **caractérisé en ce qu'**il comprend l'application, sur le cuir chevelu, d'au moins un polysaccharide sulfaté tel que défini selon l'une des revendications 1 à 4 ou d'une composition cosmétique contenant ledit polysaccharide sulfaté.

6. Procédé selon la revendication précédente, **caractérisée en ce que** le polysaccharide sulfaté est présent dans une composition cosmétique en une teneur allant de 0,01 à 20 % en poids, par rapport au poids total de la composition cosmétique.

## Patentansprüche

1. Kosmetische Verwendung von sulfatiertem Polysaccharid, ausgewählt aus Dextransulfat, den von der Rotalge Porphyridium sp. stammenden sulfatierten Polysacchariden, als Wirkstoff, um Schuppen der Kopfhaut vorzubeugen und/oder zu behandeln.

2. Verwendung nach Anspruch 1 zur Behandlung von Hautzuständen in Zusammenhang mit der Proliferation von Hefen der Gattung Malassezia auf der Kopfhaut.

3. Verwendung nach einem der vorhergehenden Ansprüche, **dadurch gekennzeichnet, dass** es sich bei dem sulfatierten Polysaccharid um Dextransulfat handelt.

4. Verwendung nach einem der Ansprüche 1 oder 2, **dadurch gekennzeichnet, dass** das sulfatierte Polysaccharid von der Rotalge Porphyridium sp. stammt.

5. Verfahren zur kosmetischen Behandlung für die Beseitigung und/oder Verringerung von Schuppen, insbesondere denjenigen, die durch die Hefen der Gattung Malassezia hervorgerufen werden, **dadurch gekennzeichnet, dass** es die Anwendung von mindestens einem sulfatierten Polysaccharid nach einem der Ansprüche 1 bis 4 oder einer kosmetischen Zusammensetzung, die dieses sulfatierte Polysaccharid enthält, auf die Kopfhaut umfasst.

6. Verfahren nach dem vorhergehenden Anspruch, **dadurch gekennzeichnet, dass** das sulfatierte Polysaccharid in einer kosmetischen Zusammensetzung in einem Gehalt von 0,01 bis 20 Gew.-% in Bezug auf das Gesamtgewicht der kosmetischen Zusammensetzung vorliegt.

## Claims

1. Cosmetic use of a sulfated polysaccharide, chosen from dextran sulfate and sulfated polysaccharides derived from the red alga Porphyridium sp., as active agent to prevent and/or treat dandruff of the scalp.

2. Use according to Claim 1 for treating dandruff conditions associated with the proliferation of yeasts of the genus Malassezia on the scalp.

3. Use according to either of the preceding claims, **characterized in that** the sulfated polysaccharide is dextran sulfate.

4. Use according to either of Claims 1 and 2, **characterized in that** the sulfated polysaccharide is derived from the red alga Porphyridium sp.

5. Cosmetic treatment process intended to eliminate and/or reduce dandruff, notably dandruff caused by yeasts of the genus Malassezia, **characterized in that** it comprises the application, to the scalp, of at least one sulfated polysaccharide as defined according to one of Claims 1 to 4, or of a cosmetic composition comprising said sulfated polysaccharide.

6. Process according to the preceding claim, **characterized in that** the sulfated polysaccharide is present in a cosmetic composition at a content ranging from 0.01 to 20% by weight relative to the total weight of the cosmetic composition.
